(19) Europäisches Patentamt
European Patent Office
Office européen des brevets

(11) EP 2 906 193 B1

(12) EUROPEAN PATENT SPECIFICATION

(45) Date of publication and mention of the grant of the patent:
**28.09.2016 Bulletin 2016/39**

(21) Application number: **13773263.2**

(22) Date of filing: **07.10.2013**

(51) Int Cl.:
***A61K 8/73*** (2006.01) ***A61K 31/728*** (2006.01)
***A61Q 19/00*** (2006.01) ***C08B 37/00*** (2006.01)

(86) International application number:
**PCT/EP2013/070814**

(87) International publication number:
**WO 2014/056841 (17.04.2014 Gazette 2014/16)**

# (54) MODIFIED HYALURONIC ACID DERIVATIVES AND USE THEREOF

MODIFIZIERTE HYALURONSÄURE-DERIVATE UND VERWENDUNG DAVON

DÉRIVÉS D'ACIDE HYALURONIQUE MODIFIÉ ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **09.10.2012 EP 12187828**

(43) Date of publication of application:
**19.08.2015 Bulletin 2015/34**

(73) Proprietor: **Sigma-Tau Industrie Farmaceutiche Riunite S.P.A.**
**00144 Rome (IT)**

(72) Inventors:
- **DI PIETRO, Antonino**
  **I-20 064 Gorgonzola (MI) (IT)**
- **CAVAZZA, Francesca**
  **I-00153 Rome (IT)**
- **CALICETI, Paolo**
  **I-35122 Padova (IT)**

(74) Representative: **Hiebl, Inge Elisabeth et al**
**Kraus & Weisert**
**Patentanwälte PartGmbB**
**Thomas-Wimmer-Ring 15**
**80539 München (DE)**

(56) References cited:
**WO-A1-00/29030**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 906 193 B1

## Description

## FIELD OF THE INVENTION

The present invention relates to hyaluronic acid derivatives and to their use in the medical and cosmetic fields, or as dietary supplements. The invention further relates to the pharmaceutical composition containing such derivatives as well as the process to obtain them.

## BACKGROUND OF THE INVENTION

For a long time researchers have devoted lots of efforts at identifying new tissue repair medical devices, either to provide new tools overcoming some disadvantages of previous ones or rather more adapted to specific tissues. In the last decade, the efforts have been even multiplied also because of an increasing demand from people desiring simply modifying their aesthetical image notably by modifying the normal course of aging.

It is well-recognized that skin is a sensible tissue that can be altered by a wide variety of natural and unnatural factors such as UV exposure, aging, smoke, burns, acne, diseases, etc...

In healthy subjects, tissue repair or tissue regeneration occurs through the healing process after an insult damaged said tissue. In the process of aging, hyaluronic acid and/or collagen production decreases in soft tissues concomitantly to an accelerated rate of degradation. Such mechanism leads to the development of depressed area such as lines, wrinkles, furrows and folds.

Currently available methods to overcome soft tissues defects include surgery (e.g., autologous or heterologous grafting) or the use of a less invasive technique involving the use of dermal fillers.

The skin is a highly organized structure consisting of three main layers, each of which having its own function. The external one called the epidermis is mainly composed of keratinocytes, and assumes a protective role from external factors such as pathogens, oxidant stress due to UV, aggression from chemicals meanwhile regulating the amount of water released from the body by trans-epidermal loss.

The medium stratum is the dermis and is a dense fibroelastic connective tissue which substantially consists of three fibrin proteins, namely collagen, elastin and reticulin together with a supporting matrix. The latter is composed of glycosaminoglycans (i.e., GAG), long chains of polysaccharides, which are able to bind a high amount of water. Together they form a gel which does not leak out of the dermis.

Finally, the inner layer called hypodermis is a fibro-fatty layer which is loosely connected to the dermis acting as an insulating layer and a protective cushion.

The dermal-epidermal junction determines the surface of the skin. Thus, a dermal-epidermal junction with anchoring structures integrity maintained folded, thereby increases the surface area of contact between the dermis and epidermis, and promote exchanges of diffusible factors between these two tissues strengthening their cohesion and improving the appearance of the skin. In cases where the anchoring structures are altered, particularly due to a deficiency in the synthesis of collagen IV, collagen VII, laminin V and/or due to aging or diseases, this causes a flattening of the dermal-epidermal junction. Indeed, it had been demonstrated that collagen IV and collagen VII are very important in wound healing process (Betz P, et al., Int. J. Legal. Med., 1992, 105, 93).

Tissue repair also contemplates chronic and/or non-healing wounds. The prevalence of such wounds increases in age-related diseases, in people affected of acquired-immune deficiency syndrome (AIDS), or in patients who have been faced to radiation after cancer intervention. Chronic wounds such as venous leg ulcers require long-term care and are very costly. Moreover, such wounds usually reappear within eighteen months of healing. Over the last four decades the concept of moist wound healing has been generally accepted giving rise to hundreds of different dressing techniques aimed at ameliorating the time and quality of healing process.

Most of the currently available dressings, apart of the traditional gauze, belong to one of the following classes: foams, hydrocolloids, hydrogels, alginates, and films; the first two representing the biggest share of the global moist wound dressings market. Hydrocolloids are most commonly made of carboxymethylcellulose, gelatin or pectin and can be combined with alginates, hyaluronates, or collagens or mixtures thereof. Dressings involving biomaterials such as collagen, hyaluronic acid, chitosan, alginates or elastin are called biological dressings.

Since collagen is the most abundant protein of extracellular matrix (ECM) collagen-based biological dressings have been extensively developed but have been progressively replaced by dressings of new generation.

It has been shown that alginate-based dressings were able to promote cellular activity such as adhesion and proliferation (Thomas S., J. Wound Care, 2000, 9, 2, 56; Thomas S., J. Wound Care, 2000, 9, 3, 115; Thomas S., J. Wound Care, 2000, 9, 4, 163).

Chitosan polysaccharide has been used in the treatment of burns and wounds due to a hypothesized stimulation of fibroblast formation and increased early phase reaction related to healing (Paul W., et. Al., Trends Biomater. Artif. Organs, 2004, 18, 18).

Finally, there exists a wide variety of hyaluronate-containing dressings, wherein hyaluronic acid has been chemically modified meanwhile maintaining its natural bio-compatibility, bio-degradability, and lack of immunogenicity. Hyaluronic acid is an endogenous polysaccharide present in elevated concentrations in the skin and connective tissue. In the skin, polymeric hyaluronic acid can bind water, forming a viscous substance that assists in hydration and turgor. Accordingly, loss of hyaluronic acid with aging is associated with increased dehydration and wrinkling of the skin. Apart from the skin, hyaluronic acid (i.e., HA) as a core component of the intracellular matrix, is also naturally found in various other tissues of the body such as tendons, muscles, cartilage, and the vitreous humor, rendering it well suited to biomedical applications targeting these tissues.

Examples of HA-based bio-material dressings are the ones wherein HA is either:

✔ unmodified as its sodium salt (e.g., ialugen, Ibsa) in the form of a cream or in gauze pads containing 4 g of ialugen for topical application.

✔ esterified via the carboxylic moieties totally or partially as described in patent EP0216453 (e.g., Hyaff®) for use in the pharmaceutical (e.g., surgical dermatology, ophthalmology, dentistry: Ballini A., et al., Int. J. Med. Sci., 2009, 6, 2, 65) or cosmetic field. These HA-esters can be extruded to produce membranes and fibers, lyophilized to obtain sponges, or processed by spray-drying, extraction, and evaporation to produce microspheres.

✔ esterified via the hydroxyl moieties (WO2004013182).

✔ linked with a further biologically active ingredient:

  o paclitaxel to prevent post surgical adhesion formation (Jackson J.K., et al., Pharm. Res., 2002, 19, 4, 411; WO02090390);

  o Ampicillin (GB2207142);

✔ cross-linked to form a molecular network with

  o a polymer of an alpha hydroxy acid such as polylactic acid (WO2006069578); 1,4-butanediol diglycidyl ether; 1,3-diaminepropane; polyfunctional epoxy derivatives (EP0161887); or can be

  o auto cross-linked between the de-acetylated amino moiety of the glycosamine residue and the carboxylic group oh the glucuronic acid moiety or between the carboxylic group oh the glucuronic acid moiety and a hydroxyl group of whatever unit (e.g., US5676964B1, Hyalobarrier® which acts as a barrier protecting and separating tissues after abdomino-pelvic surgery therefore avoiding adhesion complications).

✔ deposed on a film/gauze.

Some dressings can further contain supplementary biologically active ingredient(s) such as antibiotic, anti-inflammatory, pain killer, or growth factors or mixture thereof. A non exhaustive list of such products can be represented by Solaraze® which is a topical gel containing 3% diclofenac in 2.5% hyaluronic acid recently approved for the treatment of actinic keratoses; or by Regranex, a gel containing a recombinant human platelet derived growth factor-BB is currently in phase III clinical trial for neuropathic diabetic ulcer.

WO2007048522 disclosed a cream composition consisting of sodium hyaluronate acid, glycine and proline and possibly lysine and leucine as being effective in promoting cell reintegration in the process of fast wound-healing.

WO2010003797 disclosed HA-based compositions of different molecular weights for the treatment of corneal wounds. It was claimed that low molecular weight HA fractions (i.e., 51 kDa and 320 kDa) enhanced the healing process meanwhile the higher molecular weight HA fractions (i.e., 1500 kDa) probably because too viscous did not promote wound healing.

WO2008015249 disclosed a compositions, preferably colloidal, made of particles of high molecular weight HA and polyamines (e.g., putrecine) for use as a filler (i.e., anti-wrinkles filler or lips filler), for the treatment of wound healing and for protecting human skin against ultraviolet (UVA) radiations, but also for protecting human skin against deleterious effects of free-radicals. The only experimental data published regarded the absorbance results of various compositions, therefore directed to be used as skin protecting compositions.

Hydrogel made notably of cross-linked HA has been also reported as a polymeric matrix useful for growing and implanting cells (e.g., cells that form cartilage, cells that form bone, muscle cells, fibroblasts, and organ cells) to the specific organs (US6129761).

Dermal fillers are well-known in the art and are usually made of collagen and/or hyaluronic acid-based derivatives. In the past, the most widely used fillers were based on bovine or human collagen and tended to last 3 to 6 months. A more recent class of fillers is based on hyaluronic acid (HA) which differ between them in terms of the cross-linking pattern of HA (i.e., type and degree), particle size and formulation. Each of these parameters have been largely studied and fine tuned to give rise to fillers purposely suited to different body areas.

To overcome HA instability and/or easy degradation by hyaluronidase, crossed-linked HA filler with improved half-life appeared in the past decade. It is generally accepted that HA-based dermal fillers having a low viscosity such as those that are lightly cross-linked and/or made up of low molecular weight have a shorter duration in the body than the ones that are highly cross-linked and/or made of high molecular weight HA. The second type of fillers derived from highly modified HA is generally preferred since said fillers do not necessitate to be injected into the patient as often as with the lower viscosity ones.

The first HA-based cross-linked dermal filler to have been approved by FDA in December 2003 is Restylane™, seven years after its approval in Europe. Restylane™, also known as non-animal stabilized hyaluronic acid (NASHA) is an injectable filler composed of hyaluronic acid having a molecular weight of approximately 1 million which has been cross-linked with a two-arm cross-linker (i.e., 1,4-butanediol diglycidyl ether (BDDE)) to form ether cross-links between the two hydroxyl groups of HA molecules. Restylane™ is especially suited to correct lines in lower face and under the eyes, as well as to increase lip size. Recent histopathological research conducted on Restylane® has shown that it stimulated synthesis of collagen I and III (Wang F., et al., Arch. Dermatol., 2007, 143, 155).

A further class of fillers is represented by the Hylaform family made of Hylan B gel. Such a family is composed by Hylaform Fine lines, Hylaform Plus and Hylaform (Inamed Corporation, California, USA) and is derived from a cross-linking process using divinyl sulfone (DVS) in which the cross-linking also occurs through the hydroxyl groups of HA thus forming sulfonyl-bis-ethyl-cross-links between HA molecules.

Another cross-linked dermal filler family HA-based is Juvederm composed of various members (i.e., Juvederm 18, Juvederm 24, Juvederm 24HV and Juvederm 30) and are HA products cross-linked by means of BDDE like Restylane. However, Juvederm are claimed to be in a homogeneous gel form rather than in particle forms. Its use is use is appropriated in mid to deep dermis for correction of moderate to severe facial wrinkles and folds, such as nasolabial folds.

Perlane® which is made of larger gel particles of hyaluronic acid than Restylane® or Juvederm™ is recommended for deeper injections. A clinical trial demonstrated that a single injection with Perlane® could maintain the effects up to six months.

In patients presenting more deeply defined facial lines and creases, the use of formulations with small particle size ingredients tend to be softer and smoother, and therefore are well adapted in regions such as the lips. Larger particles have more structure, and are best suited for deep folds such as the nasolabial creases.

In aging skin it has been shown that a decrease in collagen VII expression, which is responsible for anchoring the basement membrane to dermal collagen fibres occurred (Chen Y.Q., et al., J. Invest. Dermatol., 1994, 102, 205). It has recently been found that a new C-xylopyranoside derivative induced skin expression of glycosaminoglycans and heparan sulphate proteoglycans (Pineau N., et al., Eur. J. Dermatol., 2008, 18, 1, 36).

However, besides all potential advantages of one dermal filler over another one claimed by the various companies, some doubt still persist regarding the scientific proof of said advantages. A review comparing the benefits/disadvantages of various HA-based dermal fillers according to their composition highlights the facts that not all presumed claimed advantages of the various fillers have been scientifically and thoroughly assessed (Alemman I.B., et al., Clin. Interv. Aging, 2008, 3, 4, 629).

Coated hyaluronic acid particles have been disclosed lately (WO2008147817). Needle injection is the preferred method to deliver fillers with minimum side effect in the target location.

A non-exhaustive list of applications in which a HA-based derivatives can be employed in the pharmaceutical field is reported underneath.

Laserskin®, an epidermal autograft composite made of autogenous keratinocytes grown on a biodegradable matrix made of 100% esterified HA (i.e., benzyl ester), has shown promising results in favoring complete ulcer healing in patients with chronic diabetic foot (Lobmann R., et al., J. Diabetes Complications, 2003, 17, 199).

A similar autograft composite has shown beneficial effect on chronic wounds healing of skin ulcers in recessive dystrophic epidermolysis bullosa patients (Wullina U., et al., J. Dermatol., 2001, 28, 4, 217).

A high molecular weight fractions of HA-containing gel (i.e., Gengivel®) has proven to be useful in the treatment of periodontal disease such as gingivitis (Jentsch H., et al., J. Clin. Periodontol., 2003, 30, 2, 159).

Merogel®, a woven nasal dressing made of Hyaff®, has proven to enhance the healing process in endonasal endoscopic dacryocystorhinostomy for primary chronic dacryocystitis (Wu W., et al., Eye, 2011, 25, 6, 746) as well as

A lyophilized ethyl ester of HA has proven useful in various ear pathologies and in the practice of otologic, otoneurosurgical and odontostomatological microsurgery, such as repair of tympanic perforations (US5503848).

A non-exhaustive list of applications in which a HA-based derivatives can be employed in the cosmetic field is reported underneath.

Lips augmentation, cellulite, wrinkles and dark circles around the eyes, wrinkles between the eyebrows horizontal forehead furrows, wrinkles in the corner of the mouth, irregularities from acne marks, nose and chin, depressed areas in the cheeks, temples, breast augmentation.

The use of L-carnitine alone or together with hyaluronic acid, in the cosmetic and medical field are already known.

US4839159 disclosed the use of L-carnitine for improving or healing skin conditions including wrinkling, dry or

peeling skin, and burns (particularly sunburn), and in healing and prevention of scar formation, particularly that caused by infection by a pathogen.

US7854939 disclosed the use in cosmetic of a gel made of a complex consisting of a polymer such as carboxy vinyl polymer (e.g., carbopol), a surfactant, and propionyl L-carnitine glycinate hydrochloride, for treating disturbances of the skin such as cellulite and wrinkles.

US7763655 disclosed the use of a topical composition having carnitine creatinate for inhibiting the formation of cellulite in skin.

WO2000029030 disclosed the use of complexes of hyaluronic acid and carnitine or an acyl derivative thereof having 2-20 carbon atoms, for cosmetic (e.g., beauty lotions or creams) and medical use (e.g., leg ulcer, dry eye syndrome). This patent application claimed a preferred complex containing the two components (i.e., HA and carnitine or one acyl derivative thereof) in weight ratios ranging from 1: 3 to 3: 1, preferably in equiponderal ratios.

In spite of the large number of products useful for treating skin disturbances in the medical and cosmetic field, it is still a perceived need to have new active ingredients useful for preventing or treating skin disturbances either from a pharmaceutical point of view or from a cosmetic point of view.

We have now surprisingly found that hyaluronic acid derivatives functionalised covalently with carnitine or alkanoyl carnitine are endowed of biological properties useful in the medical and cosmetic fields, and as dietary supplements. Said derivatives have demonstrated to enable regeneration of body's own collagen.

## DESCRIPTION OF THE INVENTION

The present invention relates to hyaluronic acid derivatives and their use in the medical and cosmetic fields, and as dietary supplements.

The invention provides with compounds of formula I

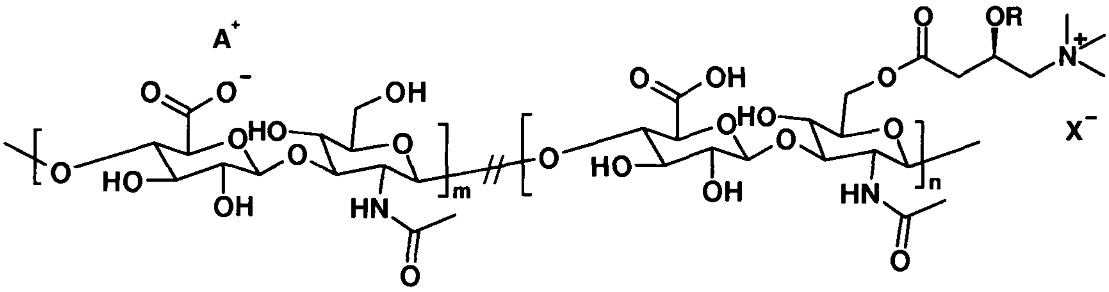

**Formula I**

comprising (m + n) repeating units; wherein m and n are integers > 0, with 70 < **(m + n)** < 5000 and with m > n; the symbol // means that two consecutive units can be either both unsubstituted, or both substituted or only one of the two is substituted;

**R** is H or an alkanoyl moiety containing from 2 to 20 carbon atoms wherein said alkanoyl moiety can be linear or branched;

**X** is Cl, Br, Ac, $MeSO_3$ or $H_2PO_4$;

**A** is H, Na, K, or TBA; or

when **X** is absent **A** is absent.

Hyaluronic acid derivatives of formula I are characterized by two parameters which are the total number of repeating units (i.e., m + n), and the substitution degree (i.e., SD). The latter even if calculated by means of HPLC, can be represented by the formula underneath.

$$SD = \left( \frac{n}{m+n} \right).$$

Preferred hyaluronic acid derivative of formula I comprises between 70 to 5000 repeating units.

More preferred hyaluronic acid derivatives of formula I have are characterized by 200 < m + n < 2000.

Even more preferred hyaluronic acid derivatives of formula I have are characterized by
400 < m + n < 1800.

Further even more preferred hyaluronic acid derivatives of formula I have are characterized by $500 < m + n < 1700$.

Furthermore, each of the above mentioned preferred hyaluronic acid derivatives of formula I have a substitution degree SD comprised between 0.01 and 0.6.

Even more preferred hyaluronic acid derivatives of formula I have a substitution degree SD comprised between 0.10 and 0.6.

The term "unit" or "repeating unit" refers either to the substituted or unsubstituted dimer constituted by D-glucuronic acid moiety and D-*N*-acetylglucosamine moiety, the latter being substituted or unsubstituted.

The expression "molar amount" and the term "equivalent" are to be construed with respect to hyaluronic acid dimer unit as represented in Figure 1.

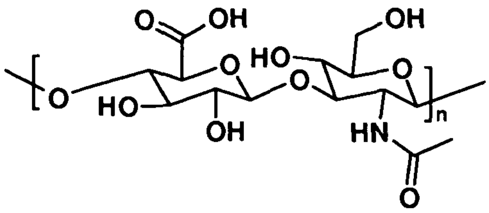

**Figure 1**

The expression "molar amount of bound carnitine" is correlated to parameter n. The expression "molar amount of polydisaccharide dimers" is correlated to parameter m.

The expression "substitution degree" and its acronym "SD" refer to the result of equation 1 underneath

$$SD = \left( \frac{\text{molar amount of bound carnitine}}{\text{molar amount of poly disaccharide dimers}} \right)$$

The expression "hyaluronic acid" is herein synonymous of hyaluronan or of its abbreviation HA. All sources of HA are useful, including bacterial and avian sources.

The expression "HA-based derivatives" refers to compounds made of chemically modified HA according to the present invention.

An embodiment of the present invention relates to compounds of formula I for use as filler agents in the cosmetic field.

In particular, the present invention relates to compounds of formula I and to their use as:

- ✓ filler for injections useful for repairing, augmenting, strengthening the tissue in need to be remodelled.
- ✓ to prevent and/or treat cellulite, scars or wrinkles; augmenting hypoplastic breasts, filling hollows of the cheeks restoring therefore a natural appearance.
- ✓ to prevent aging.

In a preferred embodiment of the invention at least one extracellular matrix component is up-regulated by the administration of compounds of formula I to the subject in need.

In a more preferred embodiment of the invention the at least one extracellular matrix is up-regulated by 5 to 90%.

In a still more preferred embodiment of the invention the at least one extracellular matrix is up-regulated by 10 to 70%.

In a still more preferred embodiment of the invention the at least one extracellular matrix is up-regulated by 10 to 70% from 6 hours after the administration and at least up to day 5 following the administration of compounds of formula I.

In a further still more preferred embodiment of the invention the at least one extracellular matrix is up-regulated is chosen from the group consisting of collagen type IV, VII, hyaluronan synthase 1 and hyaluronan synthase 2.

In a further still more preferred embodiment of the invention the at least one extracellular matrix is up-regulated is chosen from collagen type IV or VII.

It is a further object of the present invention a compound of formula I for use in restoring or maintaining activities of skin elasticity.

A further embodiment of the present invention relates to compounds of formula I for use in the medical field.

In particular, the present invention relates to compounds of formula I and to their use as:

✔ dietary supplement and medicament, for the prevention and/or treatment of disturbances of the skin, joints, arthrosis, Crohn's disease, ulcerous recto-colitis and diseases of the eye;
✔ biological dressing useful for the treatment of acute and/or chronic wounds and/or non-naturally healing wounds. A non-exhaustive list of factors that can lead to such types of wounds are burns; irradiation (either during a radiotherapy therapy or exposure to sun light); abrasions; cuts; lacerations; gunshot; diseases such as ulcers (e.g., leg and vein ulcers), notably the ones derived from diabetes; dry eye syndrome; surgery like caesarean;
✔ medical device to correct urological disorders such as urinary incontinence, gastric liquid reflux; or to repair bones, cartilage or muscle lesions;

It is a further object of the present invention a compound of formula I for use in supporting the fibrous matrix layer of tissue beneath the skin.
It is a further object of the present invention compounds of formula I for use as food supplement or as medicament, for the prevention and/or treatment of disturbances of the joints, musculoskeletal discomfort due to osteoarthritis or fibromyalgia, synovitis, gonarthrosis, Crohn's disease, ulcerous recto-colitis and diseases of the eye such as dry eye syndrome.
For cosmetic or pharmaceutical use, the compounds of formula I according to the present invention can be suitably administered orally or parenterally, in the form of liquid, semiliquid, cream, solid, in liposomes or lotion. A non limiting way of parenteral administration is: topically, intradermally, intra-articularly, or in any other parenteral suitable way well known in the art.
As a food supplement, the compounds of formula I according to the present invention can be suitably administered orally.
For ophthalmic use, the compounds of formula I according to the present invention can be suitably administered orally; or in the form of eye drops, gel or ointment to be applied topically to the eye.
According to the present invention the parenteral way of administration of the compounds of formula I includes, and is not limited to, the topical and parenteral way of administration in any part of the body in need to be treated.
According to the present invention, compounds of formula I in the form of cosmetic or pharmaceutical composition, can be administered parenterally, in a dose of from 0.1 to 30% by weight or volume, preferably from 1 to 20% by weight or volume, most preferably from 2 to 10% by weight or volume of active ingredient, optionally in admixture with one or more suitable customary auxiliary agents or further active ingredients.
According to the present invention compounds of formula I in the form of cosmetic, food supplement or pharmaceutical composition, can be administered orally in a dose of from 0.2 to 200 mg/day, preferred dose is 2-100 mg/day, the most preferred dose is about 25-50 mg/day.
The compounds of formula I for oral ingestion can be enterically coated to survive the stomach acid and to pass into the small intestine where it will absorbed.
The pharmaceutical compositions of the present invention may further comprise one or more of the following ingredients:

a) a pharmaceutically acceptable surfactant such as a stabilizing agent, a bulking agent, a cryo-protectant, a lyoprotectant, an additive, a vehicle, a carrier, a diluent, or an auxiliary. Said surfactant are well-known to the skilled person and are reported in any of the following handbooks: Pharmaceutical Dosage Forms and Drug Delivery Systems (Ansel H.C., et al., eds., Lippincott Williams & Wilkins Publishers, 7th ed. 1999**);** Remington: The Science and Practice of Pharmacy (Gennaro A.R., ed., Lippincott, Williams & Wilkins, 20th ed. 2000**);** Goodman & Gilman's The Pharmacological Basis of Therapeutics (Hardman J.G., et al., ed., McGraw-Hill Professional, 10th ed. 2001**);** and Handbook of Pharmaceutical Excipients (Rowe R.C., et al., APhA Publications, 4th edition 2003**),** and
b) at least one active ingredient useful for the prevention or treatment of disturbances of the skin selected from:

- agents supporting the microcirculation which include, but are not limited to, extracts of Gingko biloba, ruscus, melilot, red vine, viburnum;
- agents for the activation of the lipolysis which include, but are not limited to, extracts of Ground ivy (Glechoma), root of Angelica, extract of Paulinia, Subdued or of the xanthic bases such as cafeine, theobromine and theophylline;

  - anti-inflammatory compounds which include, but are not limited to, rosmarinic acid, glycyrrhizinate derivatives, alpha bisabolol, azulene and derivatives thereof, asiaticoside, sericoside, ruscogenin, escin, escolin, quercetin, rutin, betulinic acid and derivatives thereof, catechin and derivatives thereof;
  - skin whitening compounds which include, but are not limited to, ferulic acid, hydroquinone, arbutine, and kojic acid;
  - antioxidants and anti-wrinkling compounds which include, but are not limited to, retinol and derivatives,

tocopherol and derivatives, salicylates and their derivatives;

- agents which improve skin penetration and efficacy of common anticellulite agents which include, but are not limited to a monocarboxylic acids comprising lactic acid, glycolic acid, mandelic acid and mixtures thereof;
- essential fatty acids (EFAs) exerting an important role in skin defence against oxidative stress, by entering in the lipid biosynthesis of epidermis and providing lipids for the barrier formation of the epidermis; preferred essential fatty acids are selected from the group consisting of linoleic acid, gamma-linolenic acid, homo-gamma-linolenic acid, columbinic acid, eicosa-(n-6,9,13)-trienoic acid, arachidonic acid, gamma-linolenic acid, timnodonic acid, hexaenoic acid and mixtures thereof; or
- a suitable sunscreen selected from the group comprising: derivatives of para amino benzoic acid (PABA); cinnamate and benzophenone derivatives such as octyl methoxy-cinnamate, 2-hydroxy-4-methoxy-benzophenone; 3-Hydroxykynurenine O-β-DL-glucoside or a derivative thereof selected from the group comprising: 3-hydroxykynurenine O- β-D-glucoside; 3-hydroxykynurenine O- β-L-glucoside; 3-hydroxykynurenine; 4-(2-amino-3-hydroxyphenyl)-4-oxobutanoic acid O- β-D-glucoside; 4-(2-amino-3-hydroxyphenyl)-4-oxobutanoic acid O-ß-DL-glucoside; 4-(2-amino-3-hydroxyphenyl)-4-oxobutanoic acid O- P-L-glucoside; the glutathione adduct of 3-HKG; or an enantiomeric derivative thereof; or mixture thereof; or salts thereof.

c) optionally at least one excipient or diluent selected from:

- thickener agents in any suitable proportion well known to the skilled in the art; exemplary thickener agent are gums such as xanthan, carrageenan, gelatin, karaya, pectin and locust beans gum; said water-based cosmetic composition can be protected;
- preservatives against the growth of microorganisms; suitable preservatives include alkyl esters of p-hydroxybenzoic acid, hydantoin derivatives, propionate salts, methyl paraben, propyl paraben, imidazolidinyl urea, sodium dehydroxyacetate benzyl alcohol, and a variety of quaternary ammonium compounds. Preservatives, if any, are added in any suitable proportion well known to the person skilled in the art;
- silicone polymers in any suitable proportion well known to the skilled in the art;
- emollients acting both as carrier, to facilitate the dispersion of the active ingredient and skin softeners; emollients may be incorporated in the cosmetic composition of the invention in any suitable proportion well known to the skilled in the art; suitable emollients may be classified under such general chemical categories as esters, fatty acids and alcohols, polyols and hydrocarbons; an example of fatty di-esters include: dibutyl adipate, diethyl sebacate, diisopropyl dimerate, propylene glycol myristyl ether acetate, diisopropyl adipate, and dioctyl succinate; an example of branched chain fatty esters include 2-ethyl-hexyl myristate, isopropyl stearate and isostearyl palmitate; an example of tribasic acid esters include triisopropyl trilinoleate, trilauryl citrate, tributirrine, and saturated or unsaturated vegetable oils; an example of straight chain fatty esters include lauryl palmitate, myristyl lactate, oleyl eurcate, stearyl oleate coco-caprylate/caprate, and cetyl octanoate; an example of fatty alcohols and acids are $C_{10}$-$C_{20}$ compounds such as cetyl, myristyl, palmitic and stearyl alcohols and acids; an example of polyols are linear and branched chain alkyl polyhydroxyl compounds, such as propylene and butylene glycol, sorbitol glycerin, as well as polymeric polyols such as polypropylene glycol and polyethylene glycol; an example of hydrocarbons are linear $C_{12}$-$C_{30}$ hydrocarbon chains such as mineral oil, petroleum jelly, squalene and isoparaffins;
- water;
- colouring agents,
- opacifiers;
- perfumes.

The topical skin treatment composition of the invention can be formulated in all the topical forms used in beauty care: lotion, fluid cream, cream or gel. The composition can be packaged in a suitable container according to its viscosity and to the intended use by the user. For example, a lotion or fluid cream can be packaged in a bottle, in a roll-ball applicator, in a capsule, patch, in a propellant-driven aerosol device or a container fitted with a pump suitable for finger operation.

When the composition is a cream, it can simply be stored in a non-deformable bottle or in a squeeze container, such as a tube or a lidded jar.

For each particular form, one has recourse to suitable excipients.

These excipients must have all usually required qualities. As examples, one can quote: the propylene glycol, the glycerin, cetyl alcohol, the polyols, the phospholipides put in liposomes or not, oils vegetated, animal, mineral, preservatives, the dampeners, the thickeners, stabilizing and emulsifying usually used.

The expression "cosmetically acceptable ingredients" according to the present invention are products which are suitable for their use in cosmetic treatments, for example those included in the INCI list drawn by the European

Cosmetic Toiletry and Perfumery Association (COLIPA) and issued in 96/335/EC "Annex to Commission Decision of 8 May 1996".

The therapeutically effective dose of the compounds of formula I to be administered can be estimated initially either in cell culture assays or in animal models, usually mice or rats.

The animal model may also be used to determine the appropriate concentration range and route of administration. Such information can then be used to determine useful doses and routes for administration in humans.

The precise effective dose for a human subject will depend upon the severity of the disease or condition state, general health of the subject, age, weight, and gender of the subject, diet, time and frequency of administration, drug combination(s), reaction sensitivities, and tolerance/response to therapy.

This implies that the dosages of the component can be determined by the expert in the sector with normal preclinical and clinical trials, or with the usual considerations regarding the formulation of a cosmetic dietetic product.

The following illustrated examples are by no means an exhaustive list of what the present invention intends to protect.

## DESCRIPTION OF THE DRAWINGS

Figure 1: it shows the amount of LDH released upon treatment of the tissue with the composition of the invention with respect to control.
Figure 2: it shows the gene expression of HAS-1 at different time points measured by qRT-PCR.
Figure 3: it shows the gene expression of HAS-2 at different time points measured by qRT-PCR.
Figure 4: it shows the gene expression of COL7A1 at different time points measured by qRT-PCR.
Figure 5: it shows the gene expression of COL4A1 at different time points measured by qRT-PCR.
Figure 6: it shows the gene expression of SPAM1 at different time points measured by qRT-PCR.
Figure 7: it shows the release of HA-Carnitine in PBS at pH 7.4.

## EXAMPLES

### EXAMPLE 1

Conversion of sodium hyaluronate to tetrabutylammonium hyaluronate (TBA-HA/CA)

**STEP A:** hyaluronic acid

300 mg of sodium hyaluronate (0.75 mmol with reference to the disaccharide unit) were dissolved in 300 ml of deionised water and eluted at a flow rate of 1 ml/min through a 4 X 40 cm column packed with Amberlite IR 120 resin (H-Form).

**STEP B:** tetrabutylammonium hyaluronate

357 μl of tetrabutylammonium hydroxide (55% solution in $H_2O$) were then added to the collected percolate containing HA of step A to yield a stoechiometric mixture of HA and TBA having a pH 7. The solution was then dialysed through a 3 kDa membrane cut-off with 5 1 of deionised $H_2O$ for 7 seven hours. Such process was repeated twice. Subsequently, the solutions were taken together and further purified by means of ultra filtration using an Amicon system with a 10kDa cut-off cellulose membrane applying a 2.5 bar nitrogen pressure. The TBA-HA thus obtained was freeze-dried to get the desired adduct as a woven-like white solid.

Fig 1: NMR spectra of TBA-HA.

Fig 2: IR spectra of TBA-HA.

### EXAMPLE 2/1

**STEP A:** (2-hydroxy-4-imidazol-1-yl-4-oxo-butyl)-trimethyl-ammonium

273 mg of carbonyl diimidazole (1.69 mmoles) were added to a solution of 500 mg of L-carnitine hydrochloride (2.53 mmoles, 1.5 eq.) in 5 ml anhydrous DMSO. The reaction mixture was stirred under a nitrogen atmosphere for 3 hours at RT, until completion of the reaction. A sample of the solution was concentrated under vacuum, and analyzed by NMR.

$^1$H NMR (400 MHz, DMSO) δ: 8.48 (m, 1H); 7.75 (m, 1H); 7.11 (m, 1H); 4.441 (m, 1H); 3.55 (m, 2H); 3.13 (m, 9H); 2.41 (m, 2H).

**STEP B:**

The solution from Step A, containing 1.69 mmol of activated carnitine, was added to a stirred solution of 1.05 g of TBA-HA (1.69 mmol) in 60 ml of anhydrous DMSO. The reaction mixture was stirred under a nitrogen atmosphere for five days. The solution was then poured in ethanol/diethyl ether (600 ml, 50/50). The resulting precipitate was filtered, and rinsed with a 1/1 ethanol/diethylether solution.

**STEP C:**

TBA-HA-CA salt obtained from Step B was dissolved in a 5% NaCl aqueous solution and submitted to tangential fluid filtration (TFF) dialysis with a cut of 5 to 10 KDa using initially 5% sodium chloride solution and then pure water. The TBA-HA-CA thus obtained was freeze-dried to get the desired adduct as a woven-like white solid.

**EXAMPLE 2/2**

The solution from Step A of example 2/1, containing 1.69 mmol of activated carnitine, was added to a stirred solution of 640 mg of HA (1.69 mmol) in 60 ml of anhydrous formamide. The reaction mixture was stirred under a nitrogen atmosphere for five days. The solution was then poured in ethanol/diethyl ether (1 l, 50:50). The resulting precipitate was filtered, and rinsed with a 1/1 ethanol/diethyleter solution, and finally and desiccated under vacuum prior to be purified according to the procedure described at example 2/1 Step C.

**EXAMPLE 2/3**

The substituted HA/CA of example 2/3 was synthesized following the procedure described at example 2/2 modifying the ration between HA and activated carnitine from 1/1 to 1:10.

**EXAMPLE 2/4**

The substituted HA/CA of example 2/4 was synthesized following the procedure described at example 2/3 modifying the ration between HA and activated carnitine from 1/1 to 1:5.

**EXAMPLE 2/5**

The substituted HA/CA of example 2/5 was synthesized following the procedure described at example 2/3 modifying the ration between HA and activated carnitine from 1/1 to 2/1.

**EXAMPLE 2/6**

The substituted HA/CA of example 2/6 was synthesized following the procedure described at example 2/3 modifying in step B the ration between HA and activated carnitine from step A, from 1 /1 to 5/1.

**EXAMPLE 3**

**STEP A:**

(3-chlorocarbonyl-2-hydroxy-propyl)-trimethyl-ammonium

A solution of L-carnitine hydrochloride (10.5 mmoles) in 800 μl of thionyl chloride (11 mmol) was stirred under a nitrogen atmosphere for 1.5 h. Then thionyl chloride was removed under reduced pressure to lead to a transparent oil. MS analysis of a sample of this crude product dissolved in MeOH demonstrated the formation of (2-hydroxy-3-methoxycarbonyl-propyl)-trimethyl-ammonium resulting from the reaction of the expected acid chloride with MeOH.

**STEP B:**

A solution of 1.08 g of (3-chlorocarbonyl-2-hydroxy-propyl)-trimethyl-ammonium chloride (5 mmol) in DMSO

(5 ml) was added to a solution of HA (379 mg, 1 mmol with reference to the disaccharide unit) in 40 ml formamide. The resulting mixture was stirred for 1 h and then poured into EtOH. The resulting precipitate was filtered and rinsed with EtOH and $Et_2O$, and subsequently desiccated under reduced pressure prior to be purified according to the procedure described at example 2/1 Step C.

**EXAMPLE 4**

Carnitine substitution degree determination

The carnitine substitution degree determination was made by means of HPLC quantitative analysis.

**Solvents and reagents**

| | |
|---|---|
| $H_2O$: | distilled and filtered through Millipore Milli-Q filters; |
| AcCN: | HPLC grade; |
| $KH_2PO_4$: | reagent grade; |

**Equipment**

Glass volumetric flasks of 1 ml and 100 ml;
Balance accurate to 0.1 mg
Ultrasonic bath
HPLC system equipped with:

- ✓ Chromatograph: Waters Alliance mod. 2690 or equivalent
- ✓ Injector system able to inject 10 μl
- ✓ UV Detector (Waters mod. 2487 or equivalent);
- ✓ Data System (Waters "Empower 2" or equivalent);
- ✓ Column: Spherisorb SCX 5μm (250*4.6 mm internal Ø.

**Chromatographic conditions**

| | |
|---|---|
| Flow rate: | 0.7 ml/min; |
| Injected volume: | 10 μl; |
| Elution mode: | isocratic; |
| Total elution time: | 25 min; |
| Column temperature: | 30°C; |
| Detector wavelength: | 205 nm. |

**Mobile phase**

50 mM $KH_2PO_4/CH_3CN$ 40/60 (v/v). A 400 ml solution of 50 mM $KH_2PO_4$ is added into 600 ml of AcCN. The pH of the resulting mixture is adjusted pH 4.2 by addition of concentrated $H_3PO_4$. Then, said solution is degassed by means of ultrasonic bath or by bubbling pure Helium.

**Sample solution preparation**

A 10 mg sample of the substituted HA-CA was dissolved in 1 ml of 0.1 N NaOH. After 30 min the solution was neutralized by addition of 1 ml of 0.1 N HCl.

**Reference solution preparation**

A 10 mg sample of L-carnitine was dissolved in 100 ml of mobile phase (i.e., 50 mM $KH_2PO_4/CH_3CN$ 40/60 (v/v)). A 1 ml sample of this solution was further diluted to hundred volumes using the same mobile phase in order to reach a concentration of 0.001 mg/ml of L-carnitine.

**Procedure**

- ✓ The chromatographic column was conditioned with the mobile phase for 60 min.
- ✓ Inject 10 μl of blank solution. The injection is replicated two times.
- ✓ Inject 10 μl of reference solution. The injection is replicated two times.
- ✓ Inject 10 μl of sample solution. The injection is replicated two times.

**Calculation method**

The amount of carnitine freed from the polymer upon NaOH hydrolysis was quantified using equation 1 underneath:

$$SD = \frac{As \cdot Wr \cdot S}{Ar \cdot Ws} \times \frac{MW[HA - CA]}{MW[CA] \cdot 100} \qquad \textbf{Equation 1}$$

wherein:

SD: substitution degree
As: peak area of carnitine in the sample solution (mean of two injections)
Ar: peak area of carnitine in the reference solution (mean of two injections)
Wr: weight of reference sample (mg)
Ws: weight of sample (mg)
S: % strength of the reference sample

**Table 2**

| Examples | TBA-HA | HA | CA-CDI | CA-C1 | SD |
|---|---|---|---|---|---|
| 2/1 | 1 | | 1 | | 0.10 |
| 2/2 | | 1 | 1 | | 0.11 |
| 2/3 | | 1 | 10 | | 0.51 |
| 2/4 | | 1 | 5 | | 0.48 |
| 2/5 | | 2 | 1 | | 0.01 |
| 2/6 | | 5 | 1 | | 0.13 |
| 3 | | 1 | 1 5 | 5 | 0.06 |

**EXAMPLE 5**

The freeze-dried HA-CA of the above-mentioned examples was suspended in a 1 l sterile aqueous sodium phosphate buffer solution (0.1 to 30%) and stirred until obtaining a gel.

**EXAMPLE 5/1**

10 g of HA-CA from example 2/2 (SD = 0.11, MW = 900 KDa) and 0.5% aqueous sodium phosphate buffer solution were used to obtain a gel containing 1% of HA-CA.

**EXAMPLE 5/2**

20 g of HA-CA from example 2/4 (SD = 0.48, MW = 900 KDa) and 1% aqueous sodium phosphate buffer solution were used to obtain a gel containing 2% of HA-CA.

**EXAMPLE 6**

Release of HA-Carnitine in PBS at pH 7.4

16.8 mg of polymer of example 2/2 were dissolved in 3.4 ml of PBS in order to obtain a solution with a final concentration of 5 mg/ml. The solution so obtained was placed in a water bath at 37 °C. The release was monitored by HPLC using the same experimental protocol as the one described at example 4.

The results as reported in Figure 7 indicate an extremely good stability at physiological pH.

**EXAMPLE 7**

In order to evaluate the effects of the pharmaceutical composition of the invention, in vitro biological testing have been conducted on the Phenion® full thickness skin model. The latter is recognized to be a human full-thickness skin equivalent. The composition of the invention (150 μl) was injected by means of a syringe in three different points of the tissue (i.e., 50 μl for each injection) between the epidermis and the dermis. The same experimental protocol was replicated with unmodified HA, and with saline solution only, meanwhile in a forth experiment run in parallel the tissue was treated topically with the saline solution only.

*Membrane integrity determination*

The determination of membrane integrity was determined by measuring the level of lactate dehydrogenase (LDH) in the extra cellular medium. Indeed, LDH is a stable cytoplasmic enzyme present in all cells. Evidence of its presence extracellularly would inevitably be the result of cell damage determining its rapid release (Korzeniewski, C., et al., J. Immunol. Methods, 1983, 64, 313). Said determination was made by means of a commercially available colorimetric kit (i.e., Cytotoxicity Detection KIT-LDH, Roche, batch 1253300) which is based on the detection of formazan salt (λ 492 nm with reference at 690 nm). The culture supernatant was collected and incubated for 20 min with the reaction mixture included in the kit at room temperature, in the dark. An increase in the amount of dead or plasma membrane damaged cells results in an increase of the LDH enzyme activity in the culture media, said increase being directly correlated to the amount of formazan formed. A standard curve using 8 concentrations of LDH: 500 mU/ml, 250, 125, 75, 62, 5, 31, 25, 15, 62, 7, and 8 mU/ml had been previously determined.

As shown in figure 1, the LDH values in respect of either group are not statistically different, meaning that a good cell integrity upon treatment with the composition of the invention demonstrating a good biocompatibility of the composition.

*Changes in gene expression level*

In order to assess the pharmacological effects of injecting a tissue with the composition of the invention, real time PCR experiments were run considering 5 different targets (i.e., HAS1, HAS/2, COL4A1, COL7A1, SPAM1 coding for hyaluronate synthase-1, hyaluronate synthase-2, collagen type IV alpha 1, collagen, type VII alpha 1, hyaluronidase respectively).

*HAS1 gene*

HAS1 gene expression was not substantially affected after injection of HA-CA until day 5 meanwhile the injection of unmodified HA provoked a strong over-expression at 36 hours followed by a rapid down-regulation at day 5 (i.e., figure 2). Such a behaviour demonstrated that unmodified HA loosed efficacy in the medium to long term meanwhile HA-CA proved to promote synthesis of new HA enabling therefore a more rapid healing process.

*HAS2 gene*

HAS2 gene expression was up-regulated at 6 h to return to a normal level thereafter. A similar trend was observed when using unmodified hyaluronic acid instead of HA-CA (i.e., figure 3).

*COL7A1 gene*

COL7A1 gene expression proved to be highly enhanced from day 5 of the experiment involving the injection of HA-CA meanwhile its level remained sensibly stable with unmodified HA (i.e., figure 4).

*COL4A1 gene*

COL4A1 gene expression was up-regulated at 6 h time point to return to normal level later on (i.e., 36 h and 5 days). It was interesting to note (i.e., figure 5) that injection of unmodified hyaluronic acid provoked a down-regulation of COL4A1 at 5 days when compared to control group, meanwhile a similar up-regulation to that provoked by HA-CA was observed at an early time point (i.e., 6 h).

*SPAM1*

Injection of unmodified hyaluronic acid led to a strong up-regulation of SPAM1 gene at 5 days meanwhile the effect of HA-CA was much less intense at the same time point and even smaller than when saline solution was used (i.e., figure 6).

## Claims

1. A hyaluronic acid derivative of Formula I

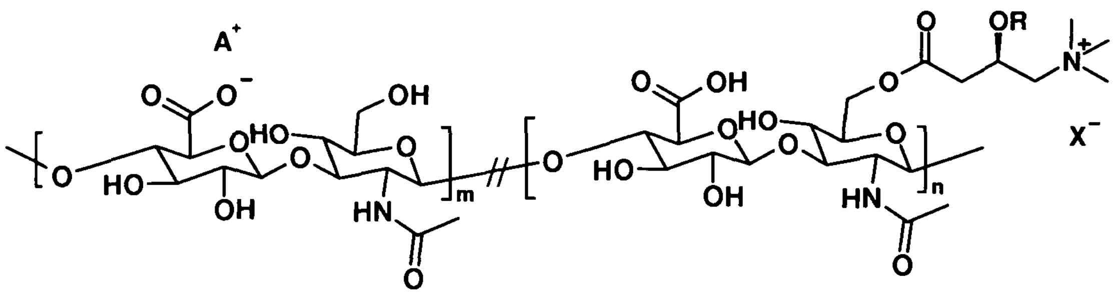

**Formula I**

comprising (m + n) repeating units; wherein m and n are integers > 0, with 70 < (m + n) < 5000 with m > n;
the symbol // means that two consecutive units can be either both unsubstituted or both substituted or only one of the two is substituted;
**R** is H or an alkanoyl moiety containing from 2 to 20 carbon atoms wherein said alkanoyl moiety can be linear or branched if it contains from 3 to 20 carbon atoms;
X is Cl, Br, Ac, $MeSO_3$ or $H_2PO_4$;
**A** is H, Na, K, or TBA; or
when **X** is absent **A** is absent.

2. The hyaluronic acid derivative according to claim 1 wherein
200 < (m + n) < 2000

3. The hyaluronic acid derivative according to claim 1which presents a substitution degree SD comprised between 0.01 and 0.60.

4. Composition comprising as an active ingredient a compound of formula I of any of claims 1 to 3, and optionally one or more diluent or excipient.

5. Composition of claim 4, enterally or parenterally administrable.

6. Composition of claim 4, for oral, topical, intradermal, intra-articular, injection or ophthalmic use.

7. Composition of claim 4, in liquid, semiliquid, cream, solid, in liposomes, or lotion form.

8. Composition of claim 5, for oral ingestion, which is enterically coated.

9. The hyaluronic acid derivative of formula I according to any of claims 1-3, for use as a filler for injections.

**10.** The hyaluronic acid derivative of formula I according to any of claims 1-3, for use as cosmetic.

**11.** The hyaluronic acid derivative of formula I according to any of claims 1-3, for use as a food supplement.

**12.** The hyaluronic acid derivative of formula I according to any of claims 1-3, for use as a medicament.

**13.** The hyaluronic acid derivative of claim 7, for use in

a) repairing defects or injury of the tissue in need to be remodelled; or
b) augmenting and strengthening soft tissue; or
c) augmenting a hypoplastic breast; or
d) correcting aphonia or dysphonia caused by paralysis of the vocal cords; or
e) the treatment of gastric fluid reflux; or
f) the treatment of defective anal sphincters; or
g) the treatment urological disorders such as vesico-ureteral reflux or urinary incontinence; or
h) repairing defects of the lips or of the hollows of the cheeks; or
i) preventing or treating cellulite or wrinkles;
j) preventing or treating disturbances of the joints, osteoarthritis, fibromyalgia, synovitis, gonarthrosis, Crohn's disease, ulcerous recto-colitis and diseases of the eye.

**Patentansprüche**

**1.** Hyaluronsäure-Derivat der Formel I

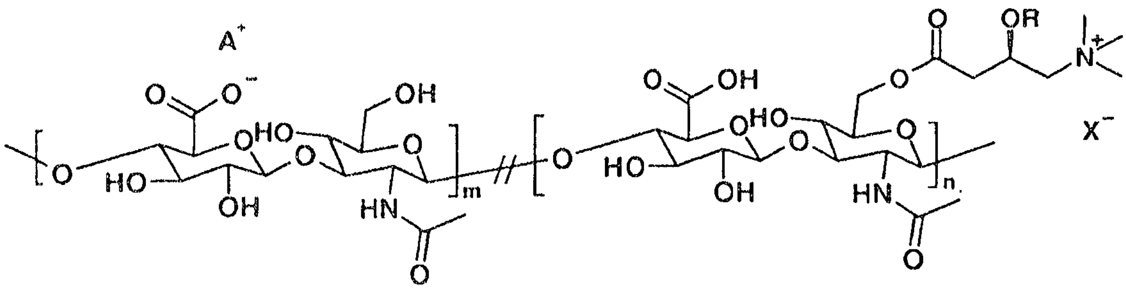

,

Formel I

das (m + n) Repetiereinheiten umfasst; wobei m und n ganze Zahlen > 0 sind, wobei 70 < (m + n) < 5000 mit m > n; wobei das Symbol // bedeutet, dass zwei aufeinanderfolgende Einheiten entweder beide unsubstituiert oder beide substituiert sein können oder dass nur eine der zwei substituiert ist;
**R** H oder eine Alkanoylgruppierung, die 2 bis 20 Kohlenstoffatome enthält, ist, wobei die Alkanoylgruppierung linear oder verzweigt sein kann, wenn sie 3 bis 20 Kohlenstoffatome enthält;
**X** für Cl, Br, Ac, $MeSO_3$ oder $H_2PO_4$ steht;
**A** für H, Na, K oder TBA steht oder
wenn **X** nicht vorhanden ist, **A** nicht vorhanden ist.

**2.** Hyaluronsäure-Derivat gemäß Anspruch 1, wobei 200 < (m + n) < 2000.

**3.** Hyaluronsäure-Derivat gemäß Anspruch 1, das einen Substitutionsgrad SD aufweist, der zwischen 0,01 und 0,60 liegt.

**4.** Zusammensetzung, umfassend als aktives Ingrediens eine Verbindung der Formel I gemäß einem der Ansprüche 1 bis 3 und gegebenenfalls ein oder mehrere Verdünnungsmittel oder Exzipientien.

**5.** Zusammensetzung gemäß Anspruch 4, die enteral oder parenteral verabreichbar ist.

**6.** Zusammensetzung gemäß Anspruch 4 zur oralen, topischen, intradermalen, intraartikulären, Injektions- oder ophthalmischen Verwendung.

**7.** Zusammensetzung gemäß Anspruch 4 in flüssiger, halbflüssiger, Creme-, Feststoff-, Liposomen- oder Lotionsform.

**8.** Zusammensetzung gemäß Anspruch 5 zur oralen Einnahme, welche enterisch beschichtet ist.

**9.** Hyaluronsäure-Derivat der Formel I gemäß einem der Ansprüche 1-3 zur Verwendung als ein Füllmittel für Injektionen.

**10.** Hyaluronsäure-Derivat der Formel I gemäß einem der Ansprüche 1-3 zur Verwendung als Kosmetikum.

**11.** Hyaluronsäure-Derivat der Formel I gemäß einem der Ansprüche 1-3 zur Verwendung als Nahrungsergänzungsmittel.

**12.** Hyaluronsäure-Derivat der Formel I gemäß einem der Ansprüche 1-3 zur Verwendung als Medikament.

**13.** Hyaluronsäure-Derivat gemäß Anspruch 7 zur Verwendung beim

a) Reparieren von Defekten oder Verletzung des Gewebes, das einer Remodellierung bedarf, oder
b) Vermehren und Verstärken von Bindegewebe oder
c) Vergrößern einer hypoplastischen Brust oder
d) Korrigieren von Aphonie oder Dysphonie, verursacht durch Paralyse der Stimmbänder, oder
e) Behandeln von gastrischem Reflux oder
f) Behandeln von mangelhaften Analsphinktern oder
g) Behandeln urologischer Störungen, zum Beispiel vesicoureteralem Reflux oder Harninkontinenz, oder
h) Reparieren von Defekten der Lippen oder der Konkavitäten der Wangen oder
i) Verhindern oder Behandeln von Cellulitis oder Falten;
j) Verhindern oder Behandeln von Störungen der Gelenke, von Osteoarthritis, Fibromyalgie, Synovitis, Gonarthrose, Crohn'scher Krankheit, ulzeröser Rektocolitis und Krankheiten des Auges.

**Revendications**

**1.** Dérivé d'acide hyaluronique de la formule I

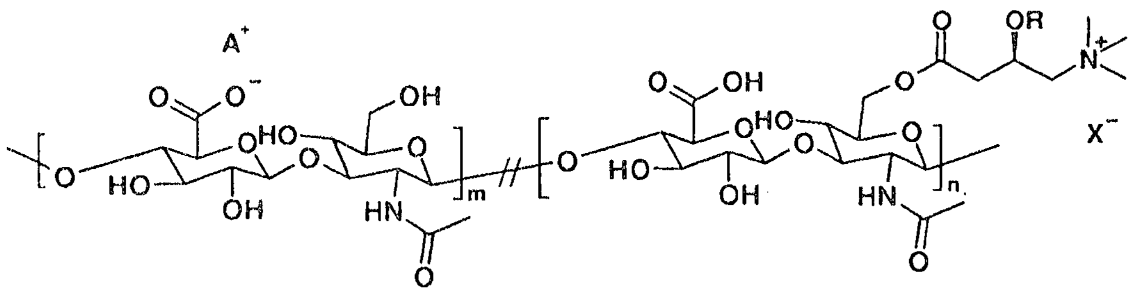

Formule I

comprenant (m+n) motifs de répétition; dans laquelle m et n sont des nombres entiers > 0, avec 70 < (m+n) < 5000 avec m > n;
le symbole // signifie que deux motifs consécutifs peuvent être substitués ou non substitués tous les deux ou seulement un seul des deux est substitué;
R représente H ou un groupement alcanoyle contenant de 2 à 20 atomes de carbone, dans lequel le groupement alcanoyle peut être linéaire ou ramifié, s'il contient de 3 à 20 atomes de carbone;
X est Cl, Br, Ac, $MeSo_3$ ou $H_2PO_4$;
A est H, Na, K ou TBA ou
lorsque X est absent, A est absent.

**2.** Dérivé d'acide hyaluronique selon la revendication 1, où 200 < (m + n) < 2000.

**3.** Dérivé d'acide hyaluronique selon la revendication 1, qui présente un degré de substitution DS compris entre 0,1 et 0, 60.

**4.** Composition comprenant comme ingrédient actif un composé de la formule I selon l'une quelconque des revendications 1 à 3 et éventuellement un ou plusieurs diluant(s) ou excipient(s).

**5.** Composition selon la revendication 4, administrable par voie entérale ou parentérale.

**6.** Composition selon la revendication 4, pour une utilisation orale, topique, intradermique, intra-articulaire, par injection ou pour une utilisation ophtalmique.

**7.** Composition selon la revendication 4 sous forme de liquide, semi-liquide, de crème, de solide, de liposomes ou sous forme de lotion.

**8.** Composition selon la revendication 5 pour la ingestion par voie orale qui est revêtue entériquement.

**9.** Dérivé d'acide hyaluronique de la formule I selon l'une quelconque des revendications 1 à 3 pour une utilisation en tant que charge pour préparations injectables.

**10.** Dérivé d'acide hyaluronique de la formule I selon l'une quelconque des revendications 1 à 3 pour utilisation comme cosmétique.

**11.** Dérivé d'acide hyaluronique de la formule I selon l'une quelconque des revendications 1 à 3 pour une utilisation comme complément alimentaire.

**12.** Dérivé d'acide hyaluronique de la formule I selon l'une quelconque des revendications 1 à 3 pour une utilisation comme médicament.

**13.** Dérivé d'acide hyaluronique selon la revendication 7 destiné à être utilisé pour

a) réparer des défauts ou une blessure du tissu qui a besoin d'être remodelé, ou
b) développer et renforcer le tissu mou, ou
c) augmenter un sein hypoplastique, ou
d) corriger l'aphonie ou la dysphonie provoquée par la paralysie des cordes vocales, ou
e) le traitement du reflux du suc gastrique, ou
f) le traitement des sphincters anaux défectueux, ou
g) le traitement des troubles urologiques tels que le reflux vésico-urétéral, ou
h) réparer des défauts des lèvres ou des creux des joues, ou
i) prévenir ou traiter la cellulite ou des rides;
j) prévenir ou traiter des troubles des articulations, l'ostéoarthrite, la fibromyalgie, la synovite, la gonarthrose, la maladie de Crohn, la rectocolite ulcéreuse et des maladies de l'oeil.

Figure 1

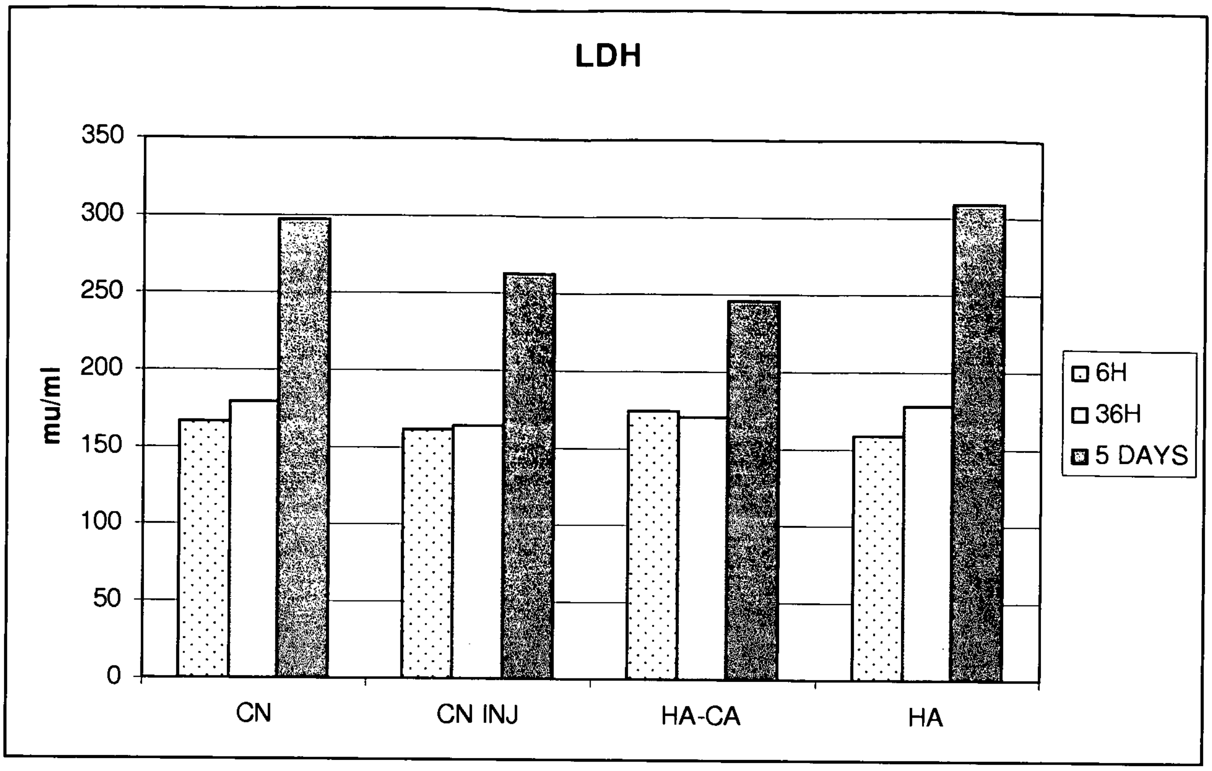


Figure 2

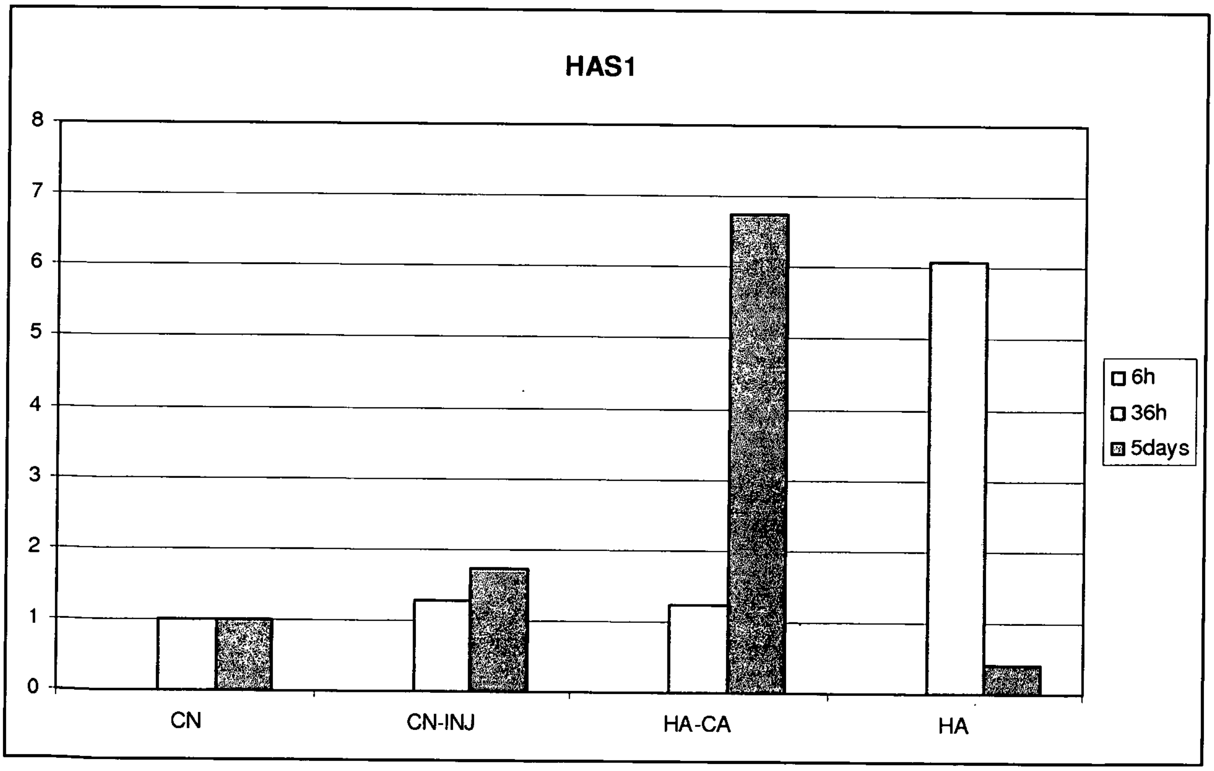

Figure 3

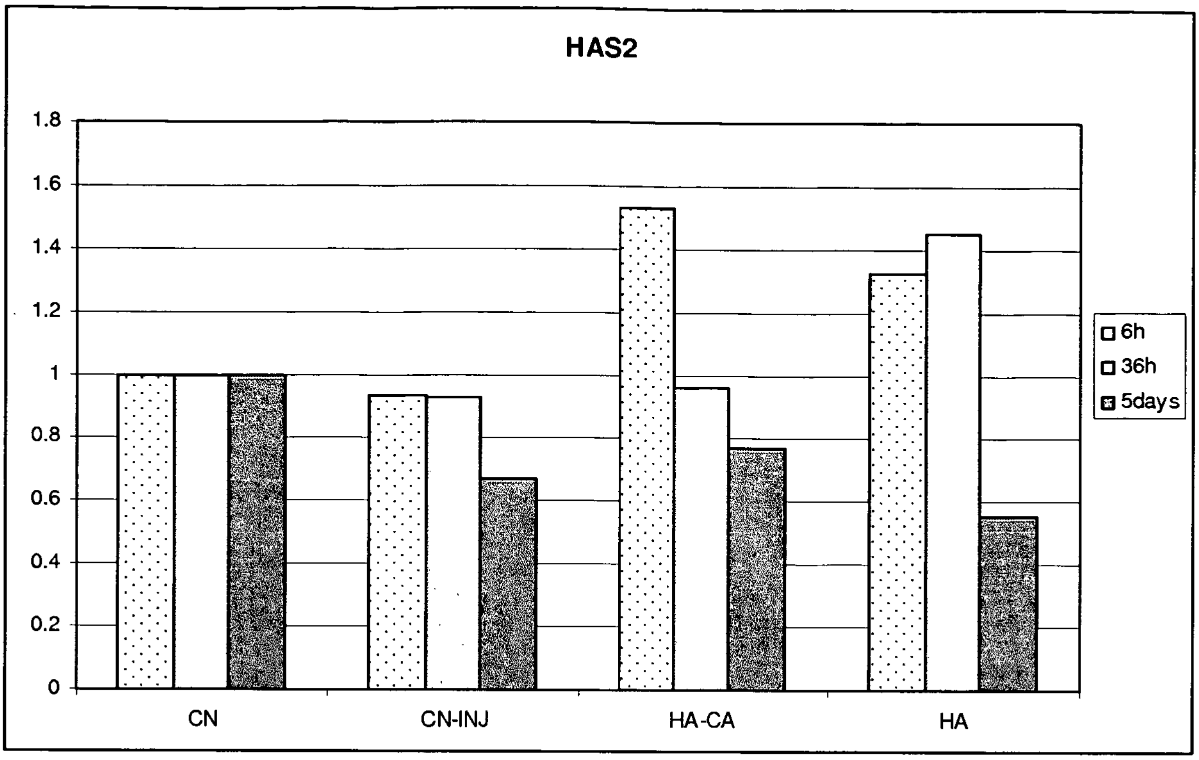


Figure 4

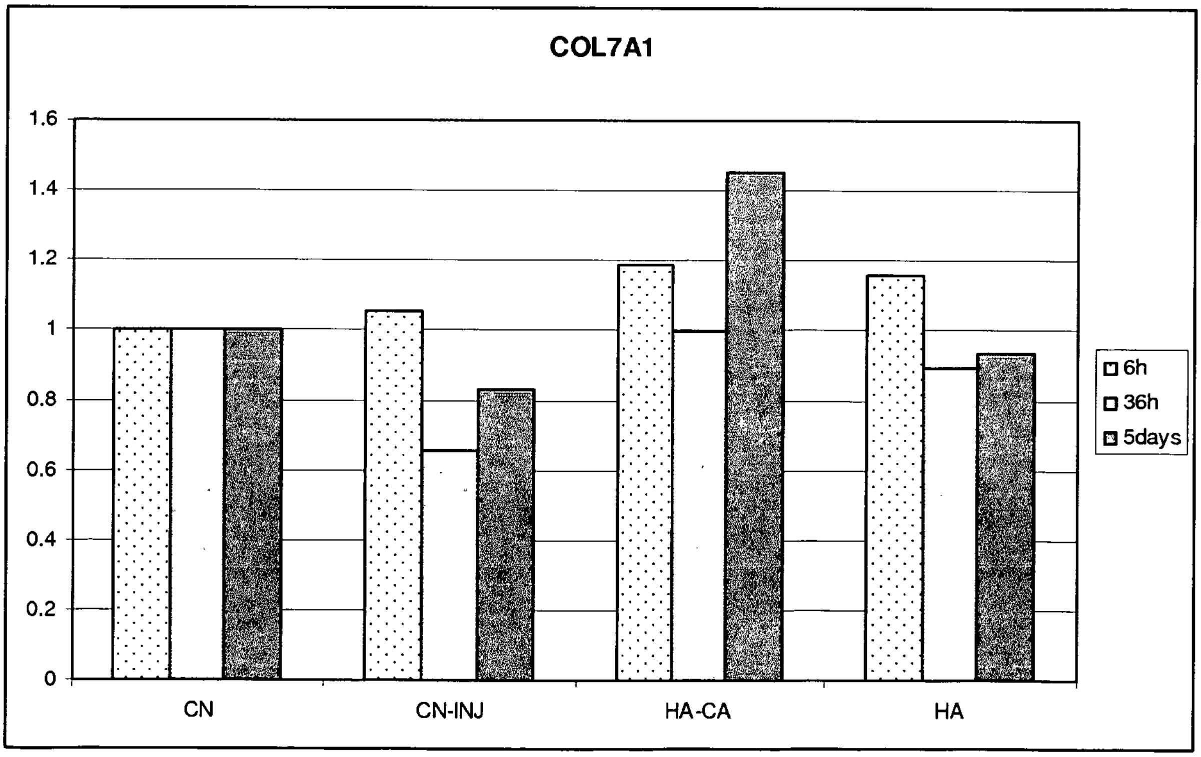

Figure 5

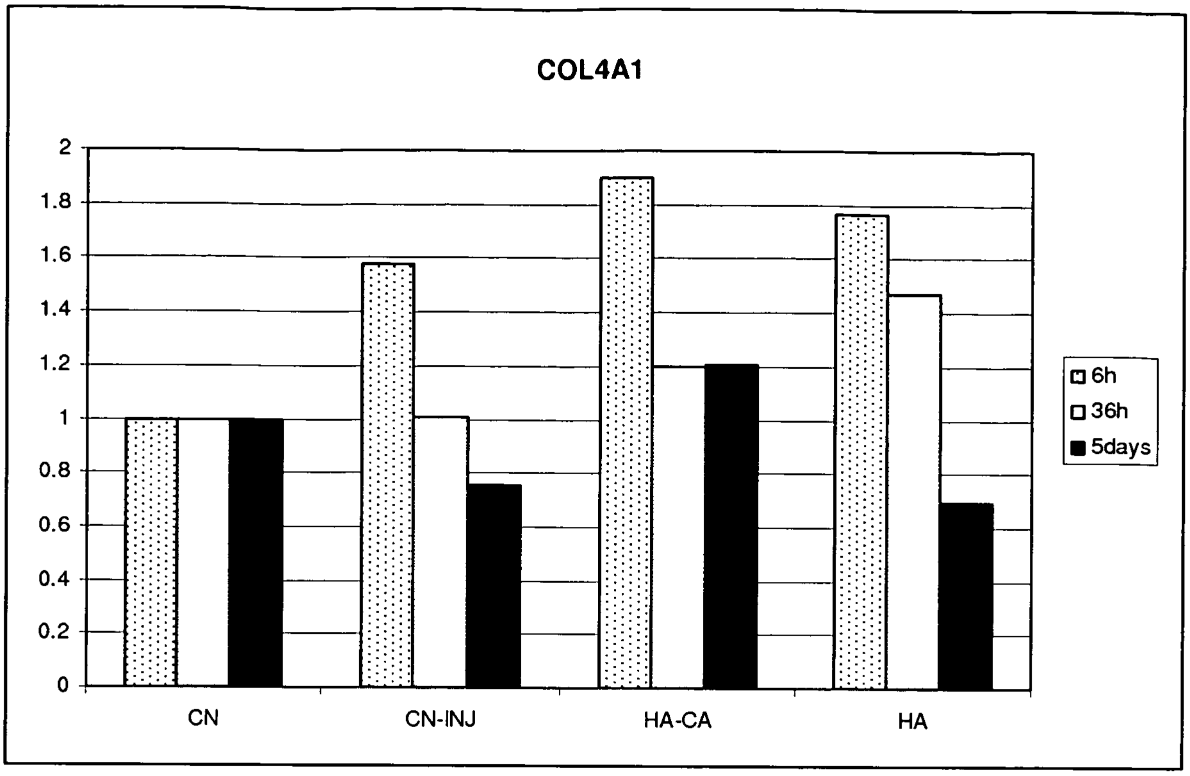


Figure 6

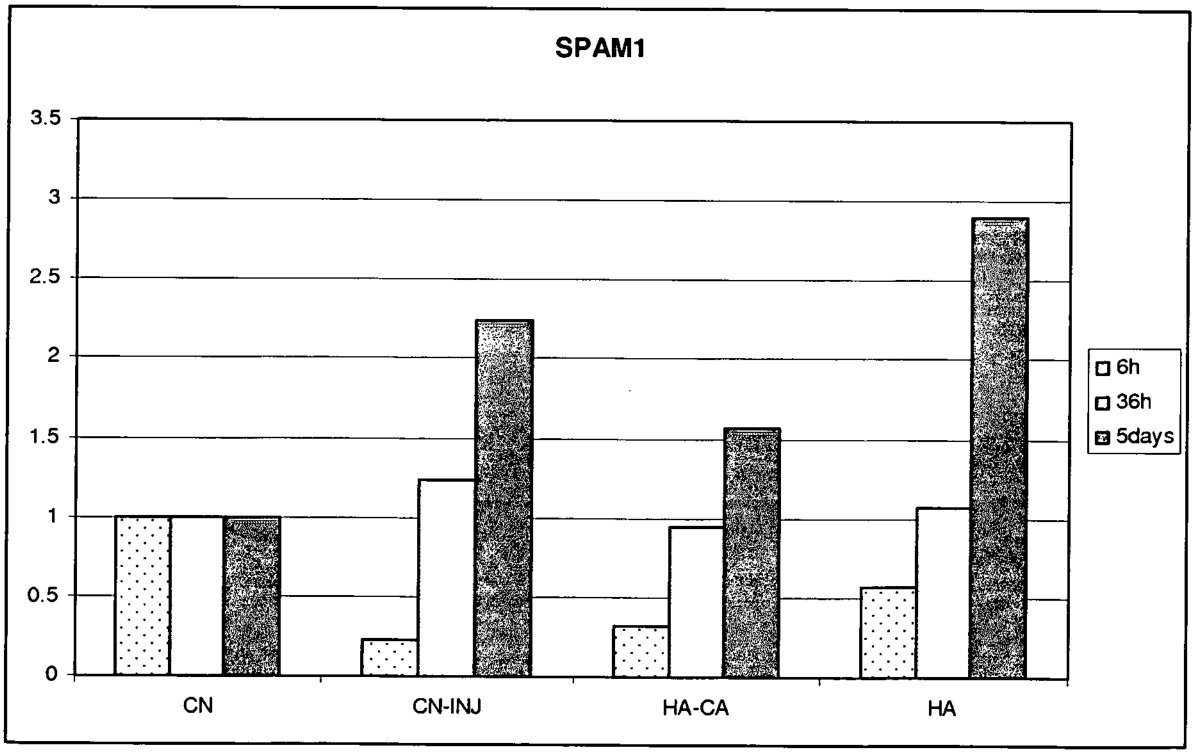

Figure 7

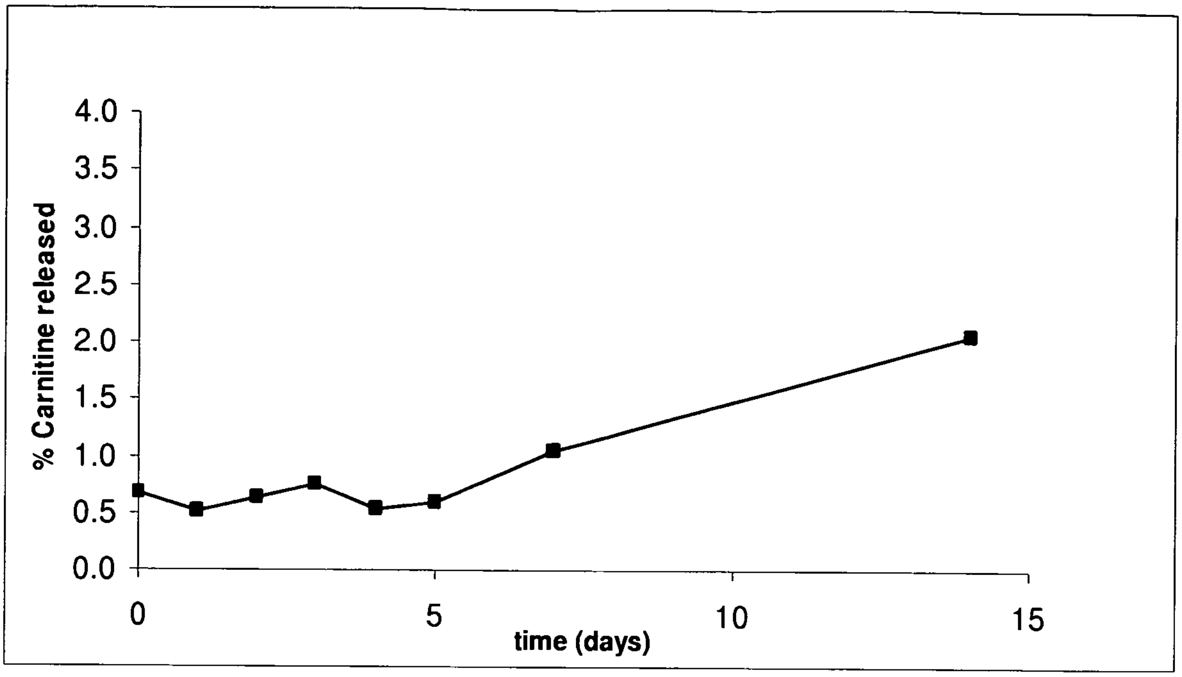

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0216453 A
- WO 2004013182 A
- WO 02090390 A
- GB 2207142 A
- WO 2006069578 A
- EP 0161887 A
- US 5676964 B1
- WO 2007048522 A
- WO 2010003797 A
- WO 2008015249 A
- US 6129761 A
- WO 2008147817 A
- US 5503848 A
- US 4839159 A
- US 7854939 B
- US 7763655 B
- WO 2000029030 A


### Non-patent literature cited in the description


- **BETZ P et al.** *Int. J. Legal. Med.,* 1992, vol. 105, 93
- **THOMAS S.** *J. Wound Care,* 2000, vol. 9, 2-56
- **THOMAS S.** *J. Wound Care,* 2000, vol. 9 (3), 115
- **THOMAS S.** *J. Wound Care,* 2000, vol. 9 (4), 163
- **PAUL W.** *Trends Biomater. Artif. Organs,* 2004, vol. 18, 18
- **BALLINI A. et al.** *Int. J. Med. Sci.,* 2009, vol. 6 (2), 65
- **JACKSON J.K. et al.** *Pharm. Res.,* 2002, vol. 19 (4), 411
- **WANG F. et al.** *Arch. Dermatol.,* 2007, vol. 143, 155
- **CHEN Y.Q. et al.** *J. Invest. Dermatol.,* 1994, vol. 102, 205
- **PINEAU N. et al.** *Eur. J. Dermatol.,* 2008, vol. 18 (1), 36
- **ALEMMAN I.B. et al.** *Clin. Interv. Aging,* 2008, vol. 3 (4), 629
- **LOBMANN R. et al.** *J. Diabetes Complications,* 2003, vol. 17, 199
- **WULLINA U. et al.** *J. Dermatol.,* 2001, vol. 28 (4), 217
- **JENTSCH H. et al.** *J. Clin. Periodontol.,* 2003, vol. 30 (2), 159
- **WU W. et al.** *Eye,* 2011, vol. 25 (6), 746
- Pharmaceutical Dosage Forms and Drug Delivery Systems. Lippincott Williams & Wilkins Publishers, 1999
- Remington: The Science and Practice of Pharmacy. Lippincott, Williams & Wilkins, 2000
- Goodman & Gilman's The Pharmacological Basis of Therapeutics. McGraw-Hill Professional, 2001
- **ROWE R.C. et al.** Handbook of Pharmaceutical Excipients. APhA Publications, 2003
- **KORZENIEWSKI, C. et al.** *J. Immunol. Methods,* 1983, vol. 64, 313